## Europäisches Patentamt
## European Patent Office
## Office européen des brevets

(19)

(11) Veröffentlichungsnummer : **0 186 895**
**B1**

(12) ## EUROPÄISCHE PATENTSCHRIFT

(45) Veröffentlichungstag der Patentschrift :
24.01.90

(51) Int. Cl.⁵ : **C 07 D301/14**, C 07 D303/04, C 07 D303/14, C 07 D303/42

(21) Anmeldenummer : 85116507.6

(22) Anmeldetag : 23.12.85

(54) Verfahren zur Epoxidation olefinisch ungesättigter Kohlenwasserstoffverbindungen mit Peressigsäure.

(30) Priorität : 31.12.84 DE 3447864

(43) Veröffentlichungstag der Anmeldung :
09.07.86 Patentblatt 86/28

(45) Bekanntmachung des Hinweises auf die Patenterteilung : 24.01.90 Patentblatt 90/04

(84) Benannte Vertragsstaaten :
AT BE CH DE FR GB IT LI NL SE

(56) Entgegenhaltungen :
DE--A-- 3 320 219
DE--B-- 1 184 343
DE--B-- 1 257 767
US--A-- 3 567 396
Die Akte enthält technische Angaben, die nach dem Eingang der Anmeldung eingereicht wurden und die nicht in dieser Patentschrift enthalten sind.

(73) Patentinhaber : Henkel Kommanditgesellschaft auf Aktien
Postfach 1100 Henkelstrasse 67
D-4000 Düsseldorf-Holthausen (DE)

(72) Erfinder : Eckwert, Klemens
Heinrich-Lersch-Strasse 54
D-4000 Düsseldorf (DE)
Erfinder : Jeromin, Lutz, Dr.
Am Bandsbusch 88
D-4010 Hilden (DE)
Erfinder : Meffert, Alfred, Dr.
Marie-Curie-Strasse 10
D-4019 Monheim (DE)
Erfinder : Peukert, Eberhard
Dürerweg 15
D-4010 Hilden (DE)
Erfinder : Gutsche, Bernhard, Dr.
Lessingstrasse 5 a
D-4010 Hilden (DE)

EP 0 186 895 B1

## Beschreibung

Die Erfindung betrifft ein Verfahren zur kontinuierlichen oder diskontinuierlichen Expoxidation von olefinische Doppelbindungen enthaltenden Kohlenwasserstoffverbindungen mit Wasserstoffperoxid und Essigsäure unter intermediärer Bildung von Peressigsäure in Gegenwart eines Katalysators. Das Verfahren eignet sich insbesondere für die Epoxidation von end- und/oder innenständig olefinisch ungesättigten und im Temperaturbereich von etwa 50 bis 100 °C unter Normaldruck flüssigen Kohlenwasserstoffverbindungen. Charakteristische Beispiele hierfür sind end- und/oder innenständige Olefine mit mehr als 12 C-Atomen, ungesättigte Alkohole — insbesondere Fettalkohole — mit etwa 8 bis 24, vorzugsweise 8 bis 18 C-Atomen sowie entsprechende Carbonsäuren, insbesondere entsprechende Fettsäuren des genannten C-Zahlbereichs und deren Ester mit ein- und/oder mehrfunktionellen Alkoholen. Das Verfahren eignet sich insbesondere auch für die Epoxidation von entsprechenden Fettsäuretriglyceriden, wobei als Beispiel das Sojaöl genannt sei.

Die Epoxidierung ungesättigter Fettsäurederivate, in erster Linie von Sojaöl, wird technisch in großem Maßstab betrieben. Es fallen dabei mit PVC verträgliche Weichmacher an, die gleichzeitig als Stabilisatoren, insbesondere gegen Wärmebeanspruchung wirken. Epoxidiertes Sojaöl ist gemäß lebensmittelrechtlichen Bestimmungen auch als Additiv für Kunststoffe zugelassen, die Kontakt mit Lebensmitteln haben.

Als Epoxidierungsmittel wird heute in der Praxis noch Perameisensäure bevorzugt, die sich in situ aus Ameisensäure und Wasserstoffperoxid erzeugen läßt, vgl. z. B. DE-A-3 320 219. Die Bildungsgeschwindigkeit der entsprechenden Persäure aus Essigsäure und Wasserstoffperoxid ist vergleichsweise geringer, scheint die Reaktionsgeschwindigkeit der Epoxidierungsreaktion mit Peressigsäure größer als bei Verwendung von Perameisensäure. Ein weiterer Vorteil der Epoxidierung mit Peressigsäure ist gegenüber der Verwendung von Perameisensäure die größere Stabilität der Peressigsäure, so daß bei geringerem Sicherheitsrisiko und neben geringeren Zersetzungsverlusten insbesondere die Persäurebildung auch außerhalb des Epoxidierungsreaktors technisch durchführbar wird. Für spezielle Epoxidationsaufgaben — vor allem bei der Epoxidierung von α-Olefinen und von ungesättigten Fettalkoholen — werden zudem mit Perameisensäure unbefriedigende Epoxidausbeuten erzielt. Dieser Mangel läßt sich aus sicherheitstechnischen Gründen beim Arbeiten mit Ameisensäure auch nicht durch Steigerung der Konzentrationen des Wasserstoffperoxids und der Ameisensäure im Reaktionsgemisch beseitigen.

Bekannt ist weiterhin die Umsetzung von Essigsäure und Wasserstoffperoxid unter Bildung von Peressigsäure über stark sauren Kationenaustauscherharzen z. B. auf Polystyrolbasis als heterogene Feststoffkatalysatoren. Geeignet sind insbesondere entsprechende gelartige und/oder makroporöse Harze, die Sulfonsäurereste als Ionenaustauschgruppen enthalten. Verwiesen wird in diesem Zusammenhang beispielsweise auf H. K. Latourette et al in J. Am. Oil Chem. S., Band 37 (1960), Seiten 559 bis 563 sowie R. J. Gall et al in J. Am. Oil Chem. S., Band 34 (1957), Seiten 161 bis 164 sowie die jeweils dort zitierte Literatur. Als geeignete Ionenaustauschkatalysatoren sind in diesen Literaturstellen beispielsweise die unter den Handelsnamen « Amberlite IR-120 », « Chempro C-20 », « Dowex 50 X » vertriebenen Produkte und andere äquivalente Harztypen genannt. Es wird dabei insbesondere vorgeschlagen, mit einer in-situ-Bildung der Peressigsäure derart zu arbeiten, daß das zu epoxidierende Kohlenwasserstofausgangsmaterial zusammen mit Wasserstoffperoxid und Essigsäure über die heterogenen Feststoffkatalysatoren geleitet bzw. mit ihnen gemeinsam in Rührwerksreaktoren bewegt wird. Diese Form der Reaktionsführung hat jedoch für die großtechnische Anwendung beträchtliche Nachteile. Die ungesättigten Kohlenwasserstoffverbindungen belegen die Katalysatorfläche und verschließen deren Poren, so daß rasch eine Inaktivierung des Katalysators für die Peressigsäurebildung auftritt. In Rührwerksreaktoren wird zusätzlich das Katalysatorkorn mechanisch zerstört, wodurch sich der zuvor beschriebene Effekt beschleunigen kann. Die thermische Belastung des Katalysators ist vergleichsweise hoch, da die Epoxidierungsreaktion im allgemeinen Temperaturen oberhalb von 50 °C, beispielsweise im Bereich bis 80 °C fordert. Unter dem Einfluß der agressiven Komponenten, $H_2O_2$ und Epoxid, wird ein vergleichsweise schnelleres Aufquellen der Ionenaustauschkatalysatoren — gegebenenfalls sogar ein partielles Auflösen — beobachtet.

Aus DE-A-1 257 767 ist schliesslich das im vorliegenden Patentanspruch 1 vor dem kennzeichnenden Teil definierte Verfahren bekannt.

Eine entscheidende Schwierigkeit bei Epoxidierungsreaktionen liegt in der Temperaturkontrolle. Die Epoxidbildung ist bekanntlich von einer beträchtlichen Wärmetönung begleitet, und es ist notwendig, diese Reaktionswärme rasch aus dem Reaktionsprodukt abzuführen, um ein unangemessenes Ansteigen der Reaktionstemperatur mit allen schädlichen Nebenfolgen zu vermeiden. Dieses Problem führt insbesondere dann zu ernsthaften Verfahrensbeschränkungen, wenn eine übermäßige Bewegung des Reaktionsgemisches zur nachträglichen leichteren Entmischung vermieden werden soll. Die beschränke Wärmeleitfähigkeit der Ölphase läßt bei außengekühlten Reaktionskolonnen lediglich beschränkte Kolonnendurchmesser zu, wenn nicht unzulässig hohe Temperaturgradienten in die Innenbereiche der durchströmten Reaktionskolonne in Kauf genommen werden sollen, vergleiche hierzu insbesondere H. K. Latourette et al aaO.

Die Erfindung geht von der Aufgabe aus, für die Epoxidation olefinisch ungesättigter Kohlenwasserstoffverbindungen unterschiedlichster Beschaffenheit mit einem wäßriges Wasserstoffperoxid, Essigsäure und Peressigsäure enthaltenden System (Sauerwasserphase) Reaktionsbedingungen und -anordnungen zu schaffen, die ein in vielfacher Weise verbessertes Arbeiten in diskontinuierlicher oder kontinuierlicher Arbeitsweise ermöglichen.

Gemäss DE-A-1 257 767 wird mit einem Kreislauf des Sauerwassers zwischen der Epoxidationsstufe und einer getrennt angeordneten Regenerationsstufe gearbeitet, in der das aus der Epoxidation abgezogene Sauerwasser bezüglich seines Peressigsäuregehalts wieder regeneriert und dann in die Epoxidation zurückgeführt wird. Mittels dieses Sauerwasserkreislaufs und der von der Epoxidation abgetrennten Sauerwasserregenerierung will nun die Erfindung eine breite Anwendungsmöglichkeit der Epoxidation mittels Peressigsäure auch gerade auf solche Kohlenwasserstoffeinsatzmaterialien ermöglichen, die nach bisherigen Verfahrensmethoden nur schwierig der Epoxidation zu unterwerfen waren. Der Sauerwasserkreislauf soll insbesondere aber auch die Problematik der Temperaturführung in der Epoxidationsstufe durch « innere Kühlung » substantiell entlasten und gleichzeitig die Regenerierung des Sauerwassers über dem sauren Ionenaustauscharz bei Temperaturen gestatten, die für diesen Regenerationsschritt und für eine lang dauernde Benutzung des hier eingesetzten heterogenen Feststoffkatalysators optimal sind.

Gegenstand der Erfindung ist dementsprechend ein Verfahren zur Epoxidation von end- und/oder innenständig olefinisch ungesättigten und im Temperaturbereich von 50 bis 100 °C unter Normaldruck flüssigen Kohlenwasserstoffverbindungen (Ölphase) durch deren Behandlung mit einer Sauerwasserphase, die in wäßriger Lösung Essigsäure, Wasserstoffperoxid und nicht mehr als 10 Geerichtsprozent Peressigsäure enthält, nachfolgende Trennung des Sauerwassers von der Ölphase, Regeneration der Peressigsäure im Sauerwasser und Rückführung des regenerierten Sauerwassers in die Epoxidation, wobei das neue Verfahren dadurch gekennzeichnet ist, daß man mit einer Sauerwasserphase arbeitet, deren Gehalt an Peressigsäure bei einem Durchgang durch die Epoxidierungsstufe um nicht mehr als 50 % — bezogen auf den Peressigsäuregehalt des eingesetzten Sauerwassers — gesenkt wird, und die Sauerwasserphase nach ihrer Abtrennung von der Ölphase kühlt, bevor man sie der Regeneration zuführt, die durch Behandlung des abgezogenen Sauerwassers — gewünschtenfalls unter Zusatz von Wasserstoffperoxid — über einem in saurer Form vorliegenden Kationenaustauscherharz als Katalysatorfestbett erfolgt, wobei die Regenerierung des Sauerwassers bei niedrigeren Temperaturen als die Epoxidationsstufe durchgeführt wird und die Epoxidation bei Normaldruck und Temperaturen im Bereich von 50 bis 80 °C erfolgt, während die Regeneration des verbrauchten Sauerwassers im Temperaturbereich von 15 bis 60 °C durchgeführt wird.

Die Regeneration der verbrauchten Peressigsäure im Sauerwasser erfolgt erfindungsgemäß bevorzugt durch Behandlung des abgezogenen Sauerwasserstroms über einem in saurer Form vorliegenden stark sauren Kationenaustauscherharz der zuvor beschriebenen Art, das als Katalysatorfestbett vorgelegt werden kann und von dem verbrauchten Sauerwasser zusammen mit den für die Regenerierung erwünschten Komponenten durchströmt wird. Als Regenerierungskomponente kommt in erster Linie Wasserstoffperoxid in Betracht. Wird es als wäßriges Konzentrat, beispielsweise als 70 %iges Wasserstoffperoxid eingesetzt, so kann die zusätzliche Einspeisung von Essigsäure und/oder Essigsäureanhydrid bei gleichzeitigem Abziehen äquivalenter Flüssigkeitsmengen aus dem Sauerwasserstrom zweckmäßig sein. Als alternative Möglichkeit bietet sich die partielle Entwässerung des aus der Epoxidierung abgezogenen Sauerwasserstroms zweckmäßigerweise vor der Einspeisung des frischen Wasserstoffperoxids an.

Erfindungsgemäß wird insbesondere mit einer vergleichsweise stark verdünnten Sauerwasserphase gearbeitet, die beim Eintritt in die Epoxidierungsstufe einen Peressigsäuregehalt im Bereich von etwa 1 bis 8 Gew.-% und vorzugsweise im Bereich von etwa 1,5 bis 6 Gew.-% besitzt. Der Wasseranteil dieser Sauerwasserphase beträgt dabei bevorzugt wenigstens etwa 40 Gew.-%, insbesondere wenigstens etwa 45 Gew.-%. Er kann 50 Gew.-% und mehr ausmachen. Der Wasserstoffperoxidgehalt liegt in der bevorzugten Ausführungsform nicht über 30 Gew.-% und kann beispielsweise im Bereich von etwa 15 bis 30 Gew.-% liegen. Der Rest ist Essigsäure, wobei die Essigsäurekonzentration im Bereich von etwa 10 bis 20 Gew.-% liegen kann. Eine besonders bevorzugte Zusammensetzung dieser Sauerwasserphase im Sinne des erfindungsgemäßen Handelns liegt in den folgenden Bereichen : etwa 15 Gew.-% Essigsäure, etwa 20 bis 25 Gew.-% Wasserstoffperoxid, etwa 1,5 bis 5 Gew.-% Peressigsäure, zum Rest Wasser.

Es ist ein weiteres bestimmendes Element für das erfindungsgemäße Verfahren, daß mit einem solchen vergleichsweise niedrig konzentrierten wäßrigen Reaktionspartner in der Epoxidierungsstufe derart gearbeitet wird, daß nur ein beschränkter Anteil der vorliegenden Peressigsäure in der Epoxidierungsreaktion abreagiert. Wie eingangs angegeben, werden nicht mehr als 50 % der im wäßrigen Einsatzmaterial vorliegenden Peressigsäure beim einmaligen Durchgang durch den Epoxidierungsreaktor umgesetzt. Tatsächlich kann es zweckmäßig sein, mit vergleichsweise niederen Umsatzzahlen im angegebenen Bereich zu arbeiten. So wird in einer bevorzugten Ausführungsform des erfindungsgemäßen Verfahrens die Reaktion in der Epoxidierungsstufe derart gelenkt, daß nicht mehr als etwa 1,5 % Peressigsäure — jetzt allerdings bezogen auf das gesamte Sauerwassergewicht — zur Reaktion kommen.

Insbesondere kann es bevorzugt sein, die Steuerung bei der Epoxidierung so zu lenken, daß etwa 0,5 bis 1 Gew.-% Peressigsäure — wiederum bezogen auf das Sauerwassergesamtgewicht — abreagieren.

Im Sinne der erfindungsgemäßen Aufgabenstellung ist es weiterhin vorgesehen, die Regenerierung des Sauerwassers bei niedrigeren Temperaturen vorzunehmen als die Epoxidation der Ölphase im Reaktor der an sich angestrebten chemischen Umsetzung. Das Sauerwasser wird dementsprechend nach Abtrennung von der Ölphase erfindungsgemäß gekühlt. Die Epoxidationsreaktion findet nach den Angaben des Standes der Technik und auch im erfindungsgemäßen Verfahren bei Temperaturen oberhalb etwa 50 °C und insbesondere im Temperaturbereich von etwa 50 bis 80 °C statt. Besonders geeignet kann der Temperaturbereich von etwa 60 bis 70 °C für die Epoxidationsreaktion sein. Demgegenüber wird die Regeneration des Sauerwassers unter Bildung von Peressigsäure bei vergleichsweise niedrigeren Temperaturen, insbesondere im Temperaturbereich von etwa 15 bis 60 °C und bevorzugt im Temperaturbereich von etwa 20 bis 40 °C durchgeführt. Durch diese Fahrweise wird — zusätzlich zur erfindungsgemäßen Maßnahme nur das Sauerwasser, nicht aber die Kohlenwasserstoffkomponenten mit dem Ionenaustauscher in Berührung zu bringen — sichergestellt, daß die Aktivität des Ionenaustauschers für die Bildung der Peressigsäure aus Wasserstoffperoxid und Essigsäure praktisch erhalten bleibt.

Als stark saure Kationenaustauscherharze eignen sich erfindungsgemäß insbesondere die eingangs genannten Ionenaustauscherharze mit Sulfonsäuregruppen, wobei neben den bereits genannten Handelsprodukten hier noch die unter den Handelsbezeichnungen « Lewatit SC 108 » oder « Lewatit SPC 108 » der Firma Bayer AG, « Amberlite MR 200 » der Firma Rohm & Haas und « Permutit RSP 120 » oder « Permutit RS-120 » der Firma Duolite' International vertriebene Ionenaustauscherharze genannt seien.

Als Kohlenwasserstoffeinsatzmaterial' für die Epoxidation (Ölphase) sind insbesondere end- und/oder innenständige Olefine mit mehr als 12 C-Atomen, ungesättigte Alkohole mit wenigstens 8 C-Atomen, vorzugsweise entsprechende Fettkohole mit beispielsweise 8 bis 18 C-Atomen sowie entsprechende ungesättigte Fettsäuren und deren Ester mit ein- und/oder mehrfunktionellen Alkoholen zu nennen. Die Einsatzmaterialien natürlichen Ursprungs — Fettsäuren bzw. Fettsäureester oder daraus hergestellte Fettkohole — können insbesondere im Gemisch ungesättigter und gesättigter Kohlenwasserstoffkomponenten eingesetzt werden. Dabei können natürlich anfallende Ausgangsmaterialien, d. h. insbesondere also Fettsäuretriglyceride etwa von der Art des Sojaöls, dem erfindungsgemäßen Verfahren unterworfen werden. Die zu verarbeitetenden Stoffe bzw. Stoffgemische sollen im Temperaturbereich der Epoxidierungsreaktion als Flüssigphase vorliegen.

Das erfindungsgemäße Verfahren läßt sich bezüglich der Epoxidationsreaktion in diskontinuierlicher Betriebsweise so einsetzen, daß in Chargenprozessen zur Epoxidierung kleinerer Mengen oder bei häufigerem Produktwechsel in jeweils der gleichen Anlage gearbeitet werden kann, beispielsweise bei der Epoxidation von end- und innenständigen Olefinen mit mehr als 12 C-Atomen oder bei der Herstellung von Epoxiden ungesättigter Fettkohole. Während sich z. B. das α-Olefin Dodecen-1 in Ausbeuten von über 80 % bisher mit Ameisensäure und Wasserstoffperoxid nur in mehrstufiger Fahrweise diskonkinuierlich epoxidieren läßt — wobei mindestens 1,7 mol/mol Doppelbindung Wasserstoffperoxid und 0,5 mol/mol Doppelbindung (DB) Ameisensäure eingesetzt werden müssen — ist dies nach dem neuen Verfahren einstufig erreichbar.

Die Epoxidierung ungesättigter Fettkohole gelang bisher wegen eintretender Nebenreaktionen nur in völlig unzureichendem Maße. Nach dem erfindungsgemäßen Verfahren können erstmals Ausbeuten von über 80 % und damit unmittelbar verwertbare Produktqualitäten epoxidierter Fettkohole hergestellt werden. Auf die erfindungsgemäße Weise ist auch bei der Epoxidierung der Olefine mit Wasserstoffperoxid und Essigsäure kein Lösungsmittel — z. B. Chloroform — erforderlich, so daß weitere Aufarbeitungsstufen entfallen.

Das erfindungsgemäße Verfahren ist insbesondere aber auch in der kontinuierlichen Verfahrensführung von beträchtlichem Vorteil. Dabei kann beispielsweise die Epoxidation in einer vorzugsweise mit Füllkörpern versehenen Reaktionskolonne durchgeführt werden, die von der Ölphase und der Sauerwasserphase durchströmt wird. Die Ölphase bildet die kontinuierliche Phase, in der die Sauerwasserphase dispers verteilt vorliegt. Durch geeignete Wahl der Füllkörper gelingt die Einstellung eines Dispersitätsgrades, der eine hinreichend rasche Reaktion zwischen Öl- und Sauerwasserphase sicherstellt, gleichwohl die leichte Abtrennung der Sauerwasserphase am Fuße der Reaktionskolonne oder nach Verlassen des Reaktionsgemisches aus der Reaktionskolonne ermöglicht. In einer bevorzugten Ausführungsform der Erfindung wird eine solche mit Füllkörpern versehene Kolonne von der Ölphase und der Sauerwasserphase im Gleichstrom von oben nach unten durchströmt. Hierbei gelingt die Steuerung der Tropfengröße in der dispersen Sauerwasserphase derart, daß Tröpfchen deutlich im sichtbaren Bereich-Durchmesser insbesondere im Bereich von 0,1 bis 5 mm, insbesondere im Bereich von 0,5 bis 3 mm — entstehen. Derartige Reaktionsgemische führen zum hinreichend raschen Ablauf der Epoxidationsreaktion in der Kolonne. Der vergleichsweise verdünnten und stark wasserhaltigen Sauerwasserphase kommt dabei nicht nur die Funktion als Reaktand mit Oxidationsvermögen zu, die disperse Sauerwasserphase dient gleichzeitig der inneren Kühlung durch Aufnahme und Abführung beträchtlicher Anteile der Reaktionswärme. Die erfindungsgemäß gewählte vergleichsweise niedrige Konzentration des Sauerwassers an Peressigsäure und der erfindungsgemäß vorgesehene beschränkte Umsetzungsgrad des Sauerwassers bei einmaligem Durchgang durch die Reaktionskolonne sind weitere Elemente zur Stabilisierung und Beherrschung des Reaktionsgeschehens.

Das Phasenverhältnis zwischen Ölphase und Sauerwasserphase wird in der bevorzugten Ausführungsform des erfindungsgemäßen Verfahrens so gewählt, daß mit höherem Raum/Zeit-Durchsatz der wäßrigen Phase im Vergleich zur Ölphase gearbeitet wird. Auch in der beschriebenen Gleichstromfahrweise beider Reaktantenströme in der Kolonne ist das unschwer möglich, da die wäßrige Sauerphase als gewichtsmäßig schwererer Reaktant durch die Ölphase hindurchfällt und somit die gewünschten Verhältniswerte der jeweiligen Mengenströme eingestellt werden können. Bevorzugt kann es sein, das Mengenverhältnis der Sauerwasserphase zur Ölphase zwischen etwa 2 bis 50 : 1 und vorzugsweise im Bereich von etwa 20 bis 40 : 1 einzustellen. Dabei ist als Sauerwasserphase das Produkt aus Kreislaufvolumen und Anzahl der Umläufe in der Reaktionszeit anzusetzen.

Nicht nur die Sauerwasserphase unterliegt der Kreislaufführung, in einer bevorzugten Ausführungsform der Erfindung — insbesondere in diskontinuierlicher Verfahrensführung — kann auch eine Kreislaufführung der Ölphase wünschenswert sein. Nach Abtrennung der Sauerwasserphase vom Reaktionsgemisch wird hier die Ölphase in den Reaktionsraum zurückgeführt.

Die Anwendung der erfindungsgemäßen Verfahrensprinzipien ist nicht auf das Arbeiten in der Reaktionskolonne mit dem beschriebenen Gleichstromprinzip beschränkt. Es kann in beliebiger Form beispielsweise auch mit Rührkesseln oder anderen Kolonnenreaktoren gearbeitet werden, wobei die Reaktionszone gewünschtenfalls auch in mehrfache Abschnitte bzw. mehrfache nacheinander geschaltete Reaktoren unter-teilt sein kann. So läßt sich z. B. Sojaöl erfindungsgemäß kontinuierlich nach dem in der DE-OS 33 20 219.2 beschriebenen Kreuzgegenstromverfahren mit Wasserstoffperoxid und Essigsäure epoxidieren, wenn dem jeweiligen Phasenabscheider einer Reaktionsstufe für die Sauerwasserphase ein Katalysatorfestbett mit dem sauren Ionenaustauscher zur Regenerierung der Peressigsäure nachgeschaltet wird.

Die Epoxidation verläuft bevorzugt im Bereich des Normaldrucks. Für die Einstellung des Gleichgewichts der Peressigsäurekonzentration in dem Sauerwasser nach dem Regenerierungsschritt über dem Ionenaustauscher-Festbett sind die Eingangskonzentrationen an Wasserstoffperoxid, Essigsäure und Wasser entscheidend. Durch die Umsetzung des Wasserstoffperoxids mit der Essigsäure zu Persäure und Wasser erhöht sich der Wasseranteil im Sauerwasser. Diese Erhöhung fällt entsprechend kleiner aus, wenn anstatt Essigsäure Essigsäureanhydrid im Rahmen des Regenerationsschrittes verwendet wird. Wird die Regeneration in der hier angedeuteten Weise unter Zusatz von Essigsäure bzw. Essigsäureanhydrid vorgenommen, so wird absatzweise oder kontinuierlich ein entsprechender Flüssigkeitsanteil dem Kreislauf der Sauerwasserphase entnommen. Die Zugabe von Essigsäure bzw. Essigsäureanhydrid entfällt, wenn der bei der Peressigsäurebildung entstandene Wasseranteil aus dem Sauerwasserkreislauf ausgeschleust werden kann und keine Verluste an Essigsäure bzw. Essigsäureanhydrid — verursacht durch deren Löslichkeit in der Epoxidphase — ausgeglichen werden müssen.

Die allgemeine Anwendbarkeit des erfindungsgemäßen Verfahrens auf ungesättigte Kohlenwasserstoffmaterialien unterschiedlichsten Ursprungs, Einsatz beliebiger Reaktoren für die Durchführung der Epoxidationsreaktion und Anwendung von Gleich- oder Gegenstromverfahren wird in den nachfolgenden Beispiele beschrieben. Das Beispiel 1 beschreibt die diskontinuierliche Epoxidation von Dodecen-1, Beispiel 2 beschreibt die kontinuierliche Epoxidation eines ungesättigten Fettalkohols bzw. Fettalkoholgemisches der Kettenlänge $C_{16/18}$ mit einer Jodzahl von 88,3 in einer Füllkörperkolonne und Beispiel 3 beschreibt schließlich die kontinuierliche Epoxidation von Sojaöl nach dem modifizierten Kreuzgegenstromverfahren der zuvor genannten Offenlegungsschrift.

Beispiel 1

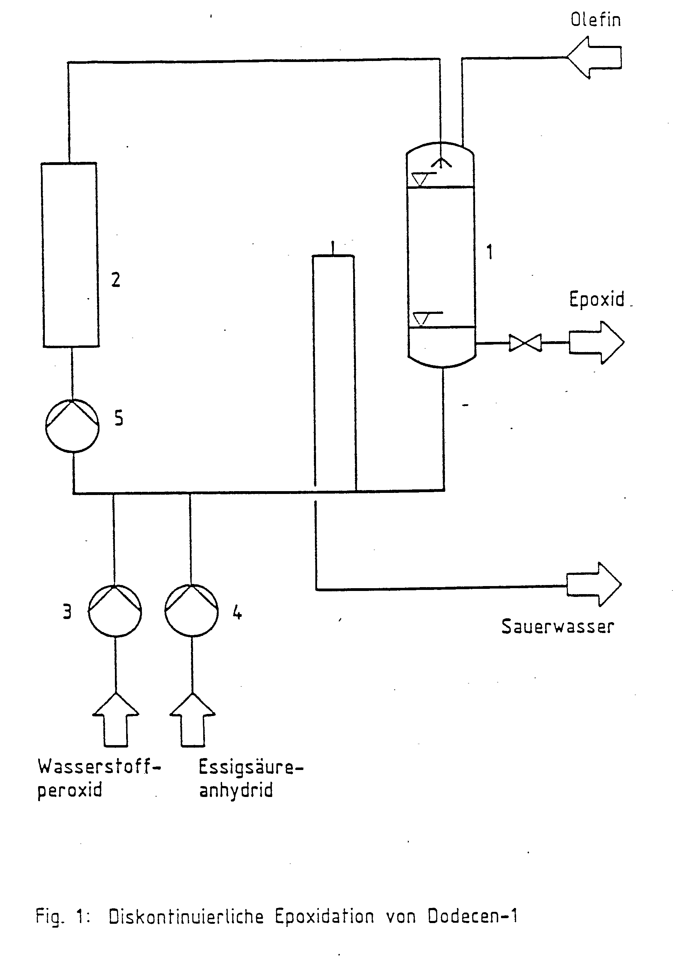

Das α-Olefin Dodecen-1 (JZ = 150) wurde entsprechend Fig. 1 mit Wasserstoffperoxid und Essigsäureanhydrid diskontinuierlich epoxidiert. Zunächst wurde 850 g Dodecen-1 im Reaktor 1 vorgelegt und der Sauerwasserkreislauf mit einer Mischung aus 22 Gewichtsprozent Wasserstoffperoxid, 25 % Essigsäure, 5 g Peressigsäure und 48 % Wasser gefüllt. Anschließend wurde die Sauerwasserkreislaufpumpe 5 in Betrieb genommen. Der Volumenstrom des umgewälzten Sauerwassers betrug 5 l/h. Nun wurden nacheinander 366 g 70 %iges Wasserstoffperoxid (1,5 mol/mol DB) mit der Pumpe 3 und 102 g Essigsäureanhydrid (0,2 mol/ mol DB) mit der Pumpe 4 in den Sauerwasserkreislauf eingespeist, während gleichzeitig eine entsprechende Menge an Sauerwasser aus dem Kreislauf verdrängt wurde. Im Behälter 2 durchströmt das umgewälzte Sauerwasser ein Ionenaustauscher-Festbett, bestehend aus 1 kg Ionenaustauscherharz des Typs « Amberlite IR 118/M » der Firma Rohm & Haas. Die Temperatur im Reaktor wurde durch indirekte Kühlung konstant auf 60 °C gehalten.

Nach 10 Stunden wurde die Pumpe 5 abgestellt und damit die Reaktion abgebrochen. Das Epoxid im Reaktor wurde abgezogen und eine neue Charge Dodecen-1 in den Reaktor gefüllt. Diese Charge konnte nun ebenfalls wie beschrieben epoxidiert werden usw. Nach Abbruch der Reaktion ergab sich jeweils eine Sauerwasserkonzentration, die, von geringfügigen Schwankungen abgesehen, der oben angegebenen Zusammensetzung des bei der ersten Charge vorgelegten Sauerwassers entsprach.

Das auf diese Weise hergestellte Epoxid besaß einen Epoxidwert von 7,2 (83,8 % Ausbeute) und eine Jodzahl von 5,1 (96,6 % Umsatz).

Beispiel 2

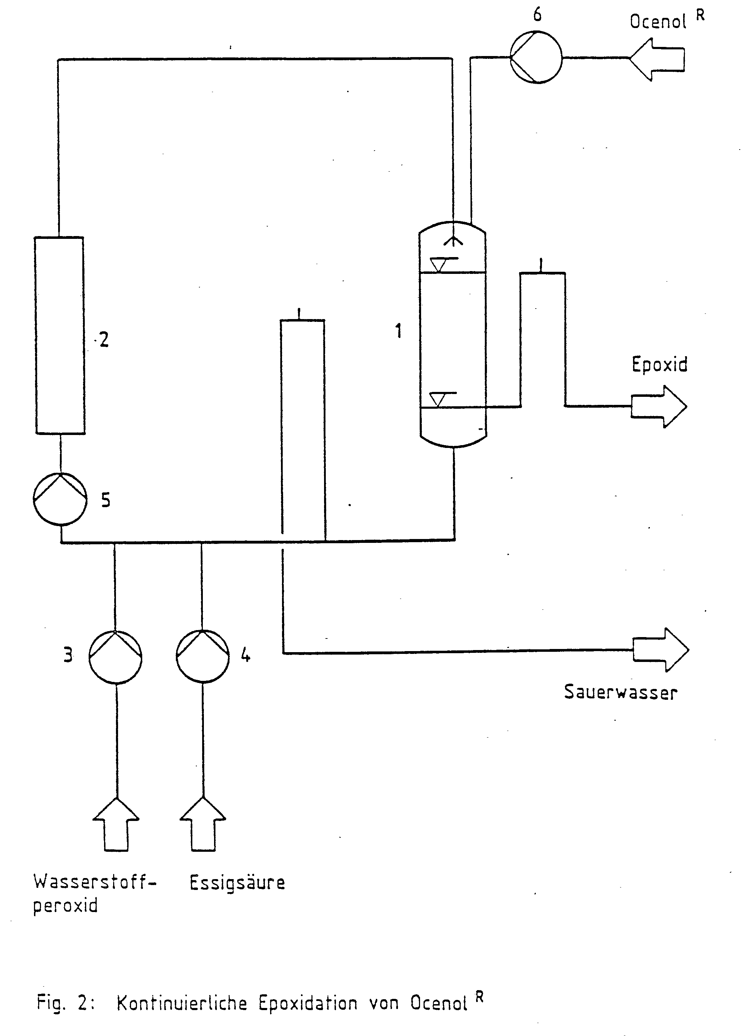

Ein ungesättigter Fettalkohol der Kettenlänge $C_{16/18}$, mit einer Jodzahl von 88,9 wurde kontinuierlich entsprechend Fig. 2 mit Wasserstoffperoxid und Essigsäure epoxidiert.

In den Reaktor 1, der ein Nutzvolumen von 1 l besaß, wurden mit der Pumpe 6 kontinuierlich 130 g/h des Fettalkohols zudosiert. Mit Hilfe der Pumpe 5 wurden 5 l/h Sauerwasser über den Ionentauscher 2 und den Reaktor 1 im Kreis gefahren, wobei im Ionentauscher 2 soviel Peressigsäure gebildet wurde wie im Reaktor 1 verbraucht wurde. In den Sauerwasserkreislauf wurden kontinuierlich 33,2 g/h (1,5 mol/mol DB) 70 %iges Wasserstoffperoxid und 13,7 g/h (0,5 mol/mol DB) Essigsäure eingespeist. Der Flüssigkeitsstand und die Phasengrenze im Reaktor 1 konnten mit belüfteten Überläufen eingestellt werden. Der Behälter 2 war mit 1 kg Ionenaustauscherharz des Typs « Lewatit SC 108 » der Firma Bayer gefüllt.

Die Temperatur im Reaktor 1 wurde durch indirekte Kühlung (Doppelmantel) konstant auf 60 °C gehalten. Um eine gute Verteilung des Sauerwassers über den Querschnitt des Reaktors zu gewährleisten, war der Reaktor mit Raschigringen aus Glas gefüllt.

Es wurde ein Epoxidwert von 4,2 (79,7 % Ausbeute) bei einer restlichen Jodzahl von 4,5 (94,9 % Umsatz) erzielt.

Beispiel 3

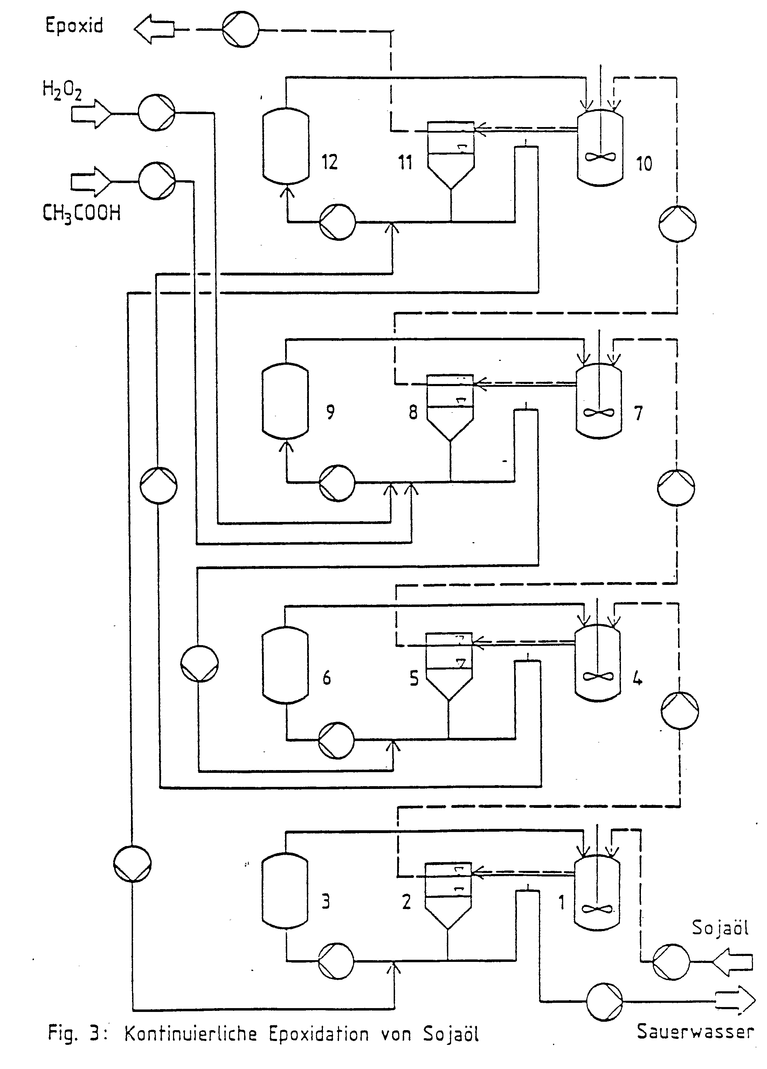

Nach dem in der DE-OS 33 20 219.2 beschriebenen Kreuzgegenstromverfahren wurde Sojaöl mit Essigsäure und Wasserstoffperoxid unter Verwendung von Ionenaustauschern zur Beschleunigung der Persäurebildung entsprechend Fig. 3 epoxidiert. Die Anlage war vierstufig, wobei jede Stufe aus einem Rührbehälter, einem Phasenabscheider und einem Ionentauscher-Festbett bestand. Der Rührbehälter und der Phasenabscheider besaßen je ein Nutzvolumen von 1 l; das Ionenaustauscher-Festbett bestand aus 500 g Ionenaustauscherharz « Lewatit SC 108 ». In jeder Stufe wurde 1 l Sauerwasser über den Ionenaustauscher im Kreis gefahren. Die Reaktionstemperaturen wurden in allen Reaktoren auf 70 °C gehalten während die Abscheider und Ionenaustauscher nicht temperiert wurden.

In den Reaktor 1 wurden 500 g/h Sojaöl kontinuierlich zudosiert. Das teilweise epoxidierte Sojaöl durchlief anschließend die Abscheider und Reaktoren in der Reihenfolge 2-4-5-7-8-10-11 (unterbrochene Linie). In den Sauerwasserkreislauf der dritten Stufe, die aus dem Reaktor 7, dem Abscheider 8 und dem Ionenaustauscher-Festbett 9 bestand, wurden kontinuierlich 149 g/h 70 %iges Wasserstoffperoxid (1,2 mol/mol DB) dosiert. Abgesehen von den Sauerwasserrückführungen durchlief das Sauerwasser die Apparate der Anlage in der Reihenfolge 9-7-8-6-4-5-12-10-11-3-1-2.

Man erhielt ein Sojaölepoxid mit einem Epoxidwert von 6,6 und einer Jodzahl von 4,8.

Patentansprüche

1. Verfahren zur Epoxidation von end- und/oder innenständig olefinisch ungesättigten und im Temperaturbereich von 50 bis 100 °C unter Normaldruck flüssigen Kohlenwasserstoffverbindungen (Ölphase) durch deren Behandlung mit einer Sauerwasserphase, die in wässriger Lösung Essigsäure, Wasserstoffperoxid und nicht mehr als 10 Gewichtsprozent Peressigsäure enthält, nachfolgende Trennung des Sauerwassers von der Ölphase, Regenerierung der Peressigsäure im Sauerwasser und Rückführung des regenerierten Sauerwassers in die Epoxidation, dadurch gekennzeichnet, daß man mit einer Sauerwasserphase arbeitet, deren Gehalt an Peressigsäure bei einem Durchgang durch die Epoxidierungsstufe um nicht mehr als 50 % — bezogen auf den Peressigsäuregehalt des eingesetzten Sauerwassers — gesenkt wird, und die Sauerwasserphase nach ihrer Abtrennung von der Ölphase kühlt, bevor man sie der Regeneration zuführt, die durch Behandlung des abgezogenen Sauerwassers — gewünschtenfalls unter Zusatz von Wasserstoffperoxid — über einem in saurer Form vorliegenden Kationenaustauscherharz als Katalysatorfestbett erfolgt, wobei die Regenerierung des Sauerwassers bei niedrigeren Temperaturen als die Epoxidationsstufe durchgeführt wird und die Epoxidation bei Normaldruck und Temperaturen im Bereich von 50 bis 80 °C erfolgt, während die Regeneration des verbrauchten Sauerwassers im Temperaturbereich von 15 bis 60 °C durchgeführt wird.

2. Verfahren nach Anspruch 1, dadurch gekennzeichnet, daß bei der Behandlung des abgezogenen Sauerwassers mit stark saure Gruppen — insbesondere Sulfonsäuregruppen — enthaltenden Ionenaustauscherharzen gearbeitet wird.

3. Verfahren nach Ansprüchen 1 und 2, dadurch gekennzeichnet, daß man mit einer beim Eintritt in die Epoxidationsstufe etwa 1 bis 8 Gewichtsprozent, vorzugsweise etwa 1,5 bis 6 Gewichtsprozent Peressigsäure enthaltenden Sauerwasserphase arbeitet, die insbesondere wenigstens etwa 40 Gewichtsprozent Wasser neben nicht mehr als 30 Gewichtsprozent Wasserstoffperoxid und zum Rest Essigsäure enthält.

4. Verfahren nach Ansprüchen 1 bis 3, dadurch gekennzeichnet, daß man mit einer Sauerwasserphase der folgenden Zusammensetzung arbeitet : etwa 15 Gewichtsprozent Essigsäure, etwa 20 bis 25 Gewichtsprozent Wasserstoffperoxid, etwa 1,5 bis 5 Gewichtsprozent Peressigsäure, Rest Wasser, und daß vorzugsweise die Reaktionsbedingungen in der Epoxidierungsstufe so gewählt sind, daß der Peressigsäuregehalt des Sauerwassers um nicht mehr als etwa 1,5 Gewichtsprozent, vorzugsweise um etwa 0,5 bis 1 Gewichtsprozent — jeweils bezogen auf das Sauerwassergewicht — gesenkt wird.

5. Verfahren nach Ansprüchen 1 bis 4, dadurch gekennzeichnet, daß die Epoxidation bei Temperaturen im Bereich von 60 bis 70 °C erfolgt, während die Regeneration des verbrauchten Sauerwassers im Temperaturbereich von 20 bis 40 °C. durchgeführt wird.

6. Verfahren nach Ansprüchen 1 bis 5, dadurch gekennzeichnet, daß die Sauerwasserphase mit höherem Raum/Zeit-Durchsatz als die Ölphase durch die Epoxidationsstufe gefahren wird, wobei das Phasenverhältnis von Sauerwasserphase zu Olefinphase bevorzugt im Bereich von etwa 2 bis 50 zu 1, insbesondere im Bereich von etwa 20 bis 40 zu 1 liegt und wobei weiterhin die Sauerwasserphase sich als Produkt aus Kreislaufvolumen und Anzahl der Umläufe in der Reaktionszeit ergibt.

7. Verfahren nach Ansprüchen 1 bis 6, dadurch gekennzeichnet, daß in diskontinuierlicher Verfahrensführung auch die Ölphase im Kreislauf geführt wird.

8. Verfahren nach Ansprüchen 1 bis 7, dadurch gekennzeichnet, daß die Epoxidation in einer vorzugsweise mit Füllkörpern versehenen Kolonne durchgeführt wird, die von der Ölphase und der Sauerwasserphase bevorzugt im Gleichstrom — insbesondere von oben nach unten — durchströmt wird.

9. Verfahren nach Ansprüchen 1 bis 8, dadurch gekennzeichnet, daß als Kohlenwasserstoffverbindungen in der Epoxidierung end- und/oder innenständige Olefine mit mehr als 12 C-Atomen, ungesättigte Alkohole, insbesondere entsprechende Fettalkohole mit wenigstens 8, insbesondere 8 bis 18 C-Atomen oder ungesättigte Fettsäuren mit insbesondere 8 bis 18 C-Atomen oder deren Ester mit ein- oder mehrwertigen Alkoholen, insbesondere auch entsprechende Triglyceride wie Sojaöl eingesetzt werden.

**Claims**

1. A process for the epoxidation of terminally and/or internally olefinically unsaturated hydrocarbon compounds (oil phase) which are liquid at 50 to 100 °C under normal pressure by treating them with an acid water phase which contains acetic acid, hydrogen peroxide and no more than 10 % by weight peracetic acid in aqueous solution, subsequently separating the acid water from the oil phase, regenerating the peracetic acid in the acid water and returning the regenerated acid water to the epoxidation stage, characterized in that the acid water phase used is one of which the peracetic acid content is reduced by no more than 50 %, based on the peracetic acid content of the acid water used, after one passage through the epoxidation stage and, after separation from the oil phase, the acid water phase is cooled before being delivered to the regeneration stage which is carried out by treatment of the acid water removed, optionally with hydrogen peroxide added, over an acidic cation exchanger resin as fixed catalyst bed, regeneration of the acid water being carried out at lower temperatures than the epoxidation stage and epoxidation being carried out under normal pressure and at temperatures in the range from 50 to 80 °C while regeneration of the spent acid water is carried out at temperatures in the range from 15 to 60 °C.

2. A process as claimed in claim 1, characterized in that treatment of the acid water removed is carried out with ion exchanger resins containing strongly acidic groups, particularly sulfonic acid groups.

3. A process as claimed in claims 1 and 2, characterized in that an acid water phase containing from about 1 to 8 % by weight and preferably from about 1.5 to 6 % by weight of peracetic acid on entering the epoxidation stage is used, this acid water phase containing in particular at least about 30 % by weight of water in addition to no more than 40 % by weight of hydrogen peroxide and, for the rest, acetic acid.

4. A process as claimed in claims 1 to 3, characterized in that the acid water phase used has the following composition: approximately 15 % by weight of acetic acid, approximately 20 to 25 % by weight of hydrogen peroxide, approximately 1.5 to 5 % by weight of peracetic acid, remainder water, and in that the reaction conditions in the epoxidation stage are preferably selected in such a way that the peracetic acid content of the acid water is reduced by no more than about 1.5 % by weight and preferably by about 0.5 to 1 % by weight, based in each case on the weight of the acid water.

5. A process as claimed in claims 1 to 4, characterized in that the epoxidation is carried out at temperatures in the range from 60 to 70 °C while regeneration of the spent acid water is carried out at temperatures in the range from 20 to 40-C.

6. A process as claimed in claims 1 to 5, characterized in that the volume/time throughput of the acid water phase through the epoxidation stage is higher than that of the oil phase, the phase ratio of acid water phase to olefin phase preferably being in the range of from about 2 to 50 : 1 and, more particularly, in the range of from about 20 to 40 : 1, the acid water phase being the product of the circulation volume and the number of circuits completed in the reaction time.

7. A process as claimed in claims 1 to 6, characterized in that, where the process is carried out non-continuously, the oil phase is also circulated.

8. A process as claimed in claims 1 to 7, characterized in that the epoxidation stage is carried out in a preferably packed column through which the oil phase and the acid water phase flow — in particular downwards — preferably in co-current.

9. A process as claimed in claims 1 to 8, characterized in that terminal and/or internal olefins containing more than 12 carbon atoms, unsaturated alcohols, particularly corresponding fatty alcohols containing at least 8 and, more particularly, from 8 to 18 carbon atoms or unsaturated fatty acids containing in particular from 8 to 18 carbon atoms or esters thereof with monohydric or polyhydric alcohols and also, and in particular, corresponding triglycerides, such as soybean oil, are used as the hydrocarbon compounds in the epoxidation stage.

**Revendications**

1. Procédé pour l'époxydation de composés hydrocarbonés à insaturation éthylénique terminale et/ou non terminale (phase huileuse) et liquides dans la plage de températures de 50 à 100 °C sous la pression normale, par traitement de ceux-ci par une phase aqueuse acide qui contient, en solution aqueuse, de l'acide acétique, du peroxyde d'hydrogène et au maximum 10 % en poids d'acide peracétique, séparation subséquente de la phase aqueuse acide d'avec la phase huileuse, régénération de l'acide peracétique dans la phase aqueuse acide, et recyclage dans l'époxydation de la phase aqueuse acide régénérée, caractérisé en ce que, l'on travaille avec une phase aqueuse acide dont la teneur en acide peracétique est réduite de 50 % au maximum — par rapport à la teneur en acide peracétique de la phase aqueuse acide de départ — par un passage à travers l'étape d'époxydation, et, après sa séparation d'avec la phase huileuse, on refroidit la phase aqueuse acide avant de l'envoyer à la régénération, laquelle est effectuée par traitement de la phase aqueuse acide soutirée — éventuellement avec addition de peroxyde d'hydrogène — sur une résine échangeuse de cations, se trouvant sous forme acide, en tant que lit solide de catalyseur, la régénération de la phase aqueuse acide étant effectuée à des températures plus basses que l'étape d'époxydation, et l'époxydation étant effectuée sous la pression normale et à des températures dans la plage de 50 à 80 °C, tandis que la régénération de la phase aqueuse acide utilisée est effectuée dans la plage de températures de 15 à 60-C.

2. Procédé selon la revendication 1, caractérisé en ce que, dans le traitement de la phase aqueuse acide soutirée, on opère avec des résines échangeuses d'ions, contenant des groupes fortement acides — en particulier des groupes sulfo.

3. Procédé selon la revendication 1 ou 2, caractérisé en ce que, l'on opère avec une phase aqueuse acide contenant, lors de son entrée dans l'étape d'époxydation, d'environ 1 à 8 % en poids, de préférence d'environ 1,5 à 6 % en poids d'acide peracétique, phase aqueuse acide qui contient en particulier au moins environ 40 % d'eau, en plus de 30 % en poids au maximum de peroxyde d'hydrogène, le reste étant constitué d'acide acétique.

4. Procédé selon l'une quelconque des revendications 1 à 3, caractérisé en ce que, l'on travaille avec une phase aqueuse acide ayant la composition suivante :
environ 15 % en poids d'acide acétique,
environ 20 à 25 % en poids de peroxyde d'hydrogène,
environ 1,5 à 5 % en poids d'acide peracétique,
le reste étant constitué d'eau,
et en ce que dans l'étage d'époxydation, on choisit de préférence les conditions réactionnelles de manière que la teneur en peracide de la phase aqueuse acide s'abaisse au maximum d'environ 1,5 % en poids, de préférence d'environ 0,5 à 1 % en poids, dans chaque cas par rapport au poids de la phase aqueuse acide.

5. Procédé selon l'une quelconque des revendications 1 à 4, caractérisé en ce que, l'on effectue l'époxydation à des températures dans la plage de 60 à 70-C, tandis que la régénération de la phase aqueuse acide utilisée est effectuée dans la plage de 20 à 40 °C.

6. Procédé selon l'une quelconque des revendications 1 à 5, caractérisé en ce que, la phase aqueuse acide est envoyée à travers l'étage d'époxydation à un débit espace/temps plus élevé que la phase huileuse, le rapport de phases de la phase aqueuse acide à la phase oléfinique se situant de préférence dans la plage d'environ 2 : 1 à 50 : 1, en particulier dans la plage d'environ 20 : 1 à 40 : 1, et la phase aqueuse acide résultant en outre du produit du volume mis en circuit par le nombre de cycles effectués pendant la durée de la réaction.

7. Procédé selon l'une quelconque des revendications 1 à 6, caractérisé en ce que, dans la mise en œuvre du procédé en mode discontinu, la phase huileuse est également mise en circuit.

8. Procédé selon l'une quelconque des revendications 1 à 7, caractérisé en ce que, l'on effectue l'époxydation dans une colonne, de préférence garnie de corps de remplissage, qui est traversée par la phase huileuse et la phase aqueuse acide de préférence en écoulement cocourant — en particulier de haut en bas.

9. Procédé selon l'une quelconque des revendications 1 à 8, caractérisé en ce que, en tant que composés hydrocarbonés, on utilise dans l'époxydation des oléfines ayant plus de 12 atomes de carbone, à double liaison terminale et/ou non terminale, des alcools insaturés, en particulier des alcools gras correspondants ayant au moins 8, en particulier de 8 à 18 atomes de carbone, on des acides gras insaturés ayant en particulier de 8 à 18 atomes de carbone ou leurs esters avec des mono — ou polyalcools, en particulier également des triglycérides correspondants, tels que l'huile de soja.

Fig. 1: Diskontinuierliche Epoxidation von Dodecen-1

6

Ocenol [R]

2

1

Epoxid

5

3

4

Sauerwasser

Wasserstoffperoxid

Essigsäure

Fig. 2: Kontinuierliche Epoxidation von Ocenol [R]

Epoxid

$H_2O_2$

$CH_3COOH$

12  11  10

9  8  7

6  5  4

3  2  1

Sojaöl

Sauerwasser

Fig. 3: Kontinuierliche Epoxidation von Sojaöl